# EUROPÄISCHE PATENTSCHRIFT

(11) **EP 1 476 750 B1**
(45) Veröffentlichungstag und Bekanntmachung des Hinweises auf die Patenterteilung: **04.02.2009**
(21) Anmeldenummer: 02795090.6
(22) Anmeldetag: 02.12.2002
(51) Int. Cl.: G01N 33/50, C07K 14/47

(54) **VERFAHREN ZUR HERSTELLUNG EINER NUKLEOSOMEN-PRÄPARATION UND DEREN VERWENDUNG ZUR IN VITRO-DIAGNOSE VON SYSTEMISCHEM LUPUS ERYTHEMASTOSUS (SLE)**
METHOD FOR PRODUCING A NUCLEOSOME PREPARATION AND USE THEREOF IN IN VITRO DIAGNOSIS OF SYSTEMIC LUPUS ERYTHEMATOSUS (SLE)
PROCEDE DE PRODUCTION D'UNE PREPARATION NUCLEOSOMIQUE ET UTILISATION DE CELLE-CI POUR LE DIAGNOSTIC IN VITRO DU LUPUS ERYTHEMATEUX SYSTEMIQUE

(30) Priorität: 15.02.2002 DE 10207735
(43) Veröffentlichungstag der Anmeldung: 17.11.2004
(73) Patentinhaber: Euroimmun Medizinische Labordiagnostika AG, 23560 Lübeck (DE)
(72) Erfinder: SUER, Waltraud, 23911 Buchholz (DE); DÄHNRICH, Cornelia, 23627 Gross Grönau (DE); SCHLUMBERGER, Wolfgang, 23627 Gross Grönau (DE); STÖCKER, Winfried, 23627 Gross Grönau (DE)
(74) Vertreter: UEXKÜLL & STOLBERG
(86) Internationale Anmeldenummer: PCT/EP2002/013629
(87) Internationale Veröffentlichungsnummer: WO 2003/069337

(56) Entgegenhaltungen:
- CLAPIER CEDRIC R ET AL: "A critical epitope for substrate recognition by the nucleosome remodeling ATPase ISWI." NUCLEIC ACIDS RESEARCH. ENGLAND 1 FEB 2002, Bd. 30, Nr. 3, 1. Februar 2002 (2002-02-01), Seiten 649-655, XP002230657 ISSN: 1362-4962
- BRUNS A ET AL: "Nucleosomes are major T and B cell autoantigens in systemic lupus erythematosus." ARTHRITIS AND RHEUMATISM. UNITED STATES OCT 2000, Bd. 43, Nr. 10, Oktober 2000 (2000-10), Seiten 2307-2315, XP002230658 ISSN: 0004-3591
- JAYASENA S D ET AL: "Nucleosome reconstitution of core-length poly(dG).poly(dC) and poly(rG-dC).poly(rG-dC)." BIOCHEMISTRY. UNITED STATES 7 FEB 1989, Bd. 28, Nr. 3, 7. Februar 1989 (1989-02-07), Seiten 975-980, XP002230659 ISSN: 0006-2960

## Beschreibung

Die vorliegende Erfindung betrifft ein Verfahren zur Herstellung einer Nukleosomen-Präparation, die sich dadurch auszeichnet, daß sie sich zum Einsatz beim Immunfluoreszenznachweis spezifischer, gegen die Nukleosomen gerichteter Antikörper in humanen Proben und damit zu einer gegenüber dem Stand der Technik spezifischeren Diagnose von Systemischem lupus erythematodes (SLE), auch bezeichnet als Lupus erythematodes disseminatus, eignet.

### Systemischer lupus erythematodes

Systemischer lupua erythematodes (SLE) ist eine vermutlich genetisch prädisponierte Autoimmunerkrankung aus der Gruppe der Kollagenosen [Yim, YS, Wakeland, EK. The genetics of lupus. Curr. Opi. Nephrol Hypertens, 2001, 10, 437-443]. Die Erkrankung beginnt meistens zwischen dem 25. und 35. Lebensjahr. Frauen erkranken wesentlich häufiger als Männer (Frauen:Männer 9:1, jährlich 50 Neuerkrankungen pro 100.000 Einwohner) [W. Olliver und D.P.M. Symmons; Autoimmunität, 1995, Spektrum Akademischer Verlag Heidelberg].

SLE ist eine systemische Erkrankung. Am häufigsten betroffen sind die Haut (Schmetterlingserythem), die Gefäße (Raynoudsyndrom), das Rippenfell (gehäuft auftretende Pleuritis) und die Gelenke (Arthritis). Vor alle die serologische Untersuchung spielt bei der Diagnose eines SLE eine wesentliche Rolle.

### Serologischer Nachweis des SLE

Charakteristisch für einen SLE ist das Auftreten autoreaktiver Antikörper, die gegen eines oder mehrere Antigene gerichtet sind. Ein Beispiel sind Antikörper gegen Doppelstrang DNS (ds-DNS), Sm, SS-A und Nukleosomen.

In eukaryontischen Zellen ist die DNS in Chromosomen organisiert. Werden die Chromosomen partiell aufgefaltet, so ähnelt die Struktur des resultierenden Chromatins einer Perlenkette. Die einzelnen Perlen nennt man Nukleosomen. Sie bestehen aus Nukleinsäuren und Proteinen, dabei enthält ein Proteinkern 8 Histonmoleküle: je 2 Exemplare der Histone H2A, H2B, H3 und H4. Um dieses Histonzentrum ist die DNS mit 146 Basenpaaren gewickelt. Verbunden sind die Nukleosomen über Linker-DNS (ca. 30-40 Bp,) wodurch die perleenkettenartige Struktur des Chromatins entsteht. Das Histon H1 befindet sich außen an den Nukleosomen in der Nähe der Linker-DNS [Luger, K, Mäder, AW, Richmond, RK, Sargent, DF, Richmond, TJ. Crystal structure of the nucleosome core particle at 2.8 Å resolution. Nature, 1997, 389, 251-260].

Chabre et al. konnten zeigen, daß nicht nur die "nackte" DNS selbst, sondern Nukleosomen ein charakteristisches Autoantigen für SLE darstellen [Chabre, H, Amoura, Z, Piette, JC, Godeau, P, Bach, JF, Koutouzov, S. Presence of nucleosome-retricted antibodies in patients with sytemic lupus erythematosus. Arthritis Rheum. 1995, 38, 1485-1491]. Es gibt Hinweise darauf, daß sich in einem frühen Stadium des SLE zunächst Antikörper gegen Nukleosomen bilden und erst zu einem späteren Zeitpunkt der Erkrankung Antikörper gegen die Bestandteile der Nukleosomen (ds-DNS, Histone) im Serum nachweisbar sind [Amoura, Z, Chabre, H, Koutouzov, S, Lotton, C, Chabrespines, A, Bach, JF, Jacob, L. Nucleosome-restricted antibodies are detected before anti-ds-DNA and/or Antihistone antibodies in serum of MRL-MP lpr/lpr and +/+ Mice, and are present in kidney eluates of lupus mice with proteinuria. Arthritis Rheum, 1994, 37, 1684-1688].

Im Stand der Technik werden Antikörper gegen Nukleosomen (ANuA) als sensitiver Marker für SLE beschrieben [Chabre et al., 1995, siehe oben]. In diesem Zusammenhang wurde ein Verfahren zur Erzeugung einer Nukleosomen-Präparation beschrieben [Amoura et al., 1994, siehe oben], bei dem nach Freisetzung der Chromatin-Bestandteile aus präparierten Zellkernen die Nukleosomen durch enzymatische Degradation dargestellt und einer Reinigung durch Saccharose-Gradienten-Zentrifugation unterzogen wurden. Der angewandte Gradient betrug 5 bis 20 %. Bislang ist es jedoch keiner Arbeitsgruppe gelungen, die Nukleosomen soweit zu reinigen, daß nur noch SLE-Patienten reagieren.

Studien zum Thema Anti-Nukleosomen-Antikörper (ANuA) zeigten, daß bei 20% bis 68% der Patienten mit progressiver stemsklerose (PSS) Antikörper gegen Nukleosomen nachweisbar sind [Dick, T, Vondegracht, MC, Franz, K, Mierau, R, Genth, E. Nachweis von anti-Nukleosomen-Antikörpern bei Patienten mit SLE, systemische Sklerose und Kontrollkollektiven. Vergleich von 4 ELISA Systemen, Rheumaklinik und Rheumaforschungsinstitut Aachen; Amoura Z, Koutouzov S, Chabre H, Cacoub P, Amoura I, Musset L, Bach JP, Piette JC.Presence of antinucleosome autoantibodies in a restricted set of connective tissue diseases: antinucleosome antibodies of the IgG3 subclass are markers of renal pathogenicity in systemic lupus erythematosus. Arthritis Rheum. 2000; 43, 76-84]. Auch bei Polymyositis lassen sich zu einem geringen Prozentsatz ANuA mit dem konventionellen Testsystem nachweisen. Bisher ist es also nicht gelungen, durch die Bestimmung der ANuA zwischen SLE und anderen Erkrankungen zu unterscheiden, dadurch ist der Wert der ANuA als diagnostischer Marker sehr stark eingeschränkt.

Das im Stand der Technik bekannte Verfahren zur SLE-Diagnose ist daher unspezifisch und erfordert die Anwendung weitergehender diagnostischer Methoden, um eine Unterscheidung zwischen SLE und PSS zu ermöglichten.

Aufgabe der vorliegenden Erfindung ist es daher, ein *in vitro-*Diagnose-Verfahren bereitzustellen, mit dem es möglich ist, systemischen lupus erythematosus spezifisch nachzuweisen und eindeutig von progressiver Systemsklerose zu unterscheiden. Die Aufgabe wird erfindungsgemäß durch eine neue Nukleosomen-Präparation und einen Assay unter Verwendung dieser Nukleosomen als Antigen gelöst.

### Kurze Darstellung der Erfindung

Im Rahmen der vorliegenden Erfindung wurde überraschenderweise festgestellt, daß sich Nukleosomen - entgegen den im Stand der Technik vorliegenden Daten und Informationen - sehr wohl als spezifischer Marker eignen, wenn man sie von Histon 1, nicht-Histon-Proteinen und dem Protein Scl-70 befreit. Diese Scl-70 freien Nukleosomen werden durch ein modifiziertes Aufarbeitungsverfahren erhalten, bei dem man nach Aufreinigung der Zellkerne und anschließendem Nuklease-Verdau aufgereinigte Nukleosomen durch Dichtezentrifugation mit einem Saccharose-Gradienten isoliert. Im Rahmen der vorliegenden Erfindung kann unerwarteterweise eine gegenüber dem Stand der Technik deutlich reinere Fraktion erhalten werden, wenn man einen Saccharose-Gradienten mit mindestens 30 (vorzugsweise mindestens 35) Gew.-% Saccharose, vorzugsweise 30 bis 50 Gew.-%, besonders bevorzugt 35 (oder 40) bis 50 Gew.-%, am meisten bevorzugt etwa 50 Gew.-% Saccharose, verwendet. Auf diese Weise wird eine Nukleosomen-Präparation isoliert, die aus Mononukleosomen besteht, die frei von Histon 1 und Scl-70-Protein und vorzugsweise auch frei von anderen nicht-Histon-Proteinen, wie z.B. HMGN, sind.

Überraschenderweise kommt es erfindungsgemäß zu keiner Kreuzreaktivität mit PSS-assoziierten Antikörpern, wenn man die Nukleosomen dieser modifizierten Aufreinigungsprozedur unterzieht, so daß die gewonnene Präparation frei von Scl-70-Protein ist.

Vorliegend bedeutet "frei von Histon 1 und Scl-70-Protein" bzw. "frei von anderen nicht-Histon-Proteinen", daß die Nukleosomen-Präparation die genannten Proteine nicht oder nur in einem so geringen Anteil enthält, der für den spezifischen SLE-Nachweis und die Unterscheidung von PSS nicht störend ist. Unter "anderen nicht-Histon-Proteinen" werden beispielsweise auch HMG (high mobility group) Proteine verstanden, die ebenfalls als Autoantigene der PSS beschrieben wurden [Rosenberg AM, Uziel Y, Krafchik BR, Hauta SA, Prokopchuk PA, Silverman ED, Laxer RM. Antinuclear antibodies in children with localized scleroderma. J Rheumatol 1995, 22, 2337-2343; Vlachoyiannopoulos PG, Boumba VA, Tzioufas AG, Seferiadis C, Tsolas O, Moutsopoulos HM. Autoantibodies to HMG-17 nucleosomal protein in patients with scleroderma. J Autoimmun 1994, 7, 193-201].

Mononukleosomen lassen sich für die vorliegenden Zwecke rein oder nahezu rein erhalten, wenn man Nukleosomen beispielsweise durch Saccharose-Gradienten-Zentrifugation unter Verwendung eines Saccharose-Gradienten von über 30 % aufreinigt und auf diese Weise von Histon H1 und weiteren Proteinen, insbesondere von einer 70 kDa Proteinfraktion, abtrennt. Dadurch werden erfindungsgemäß Nukleosomen erhalten, die gegenüber den im Stand der Technik bekannten Präparationen eine erhöhte Reinheit aufweisen und im ELISA zu keiner Kreuzreaktivität mit PSS-assoziierten Antikörpern führen. Durch die Verwendung dieser Mononukleosomen in der in vitro-Diagnostik, z.B. als Antigen im ELISA, wird sowohl ein sensitives als auch hoch spezifisches Testsystem für die serologische Bestimmung des LED erhalten.

### Detaillierte Beschreibung der Erfindung

Gegenstand der vorliegenden Erfindung ist somit eine Nukleosomen-Präparation, die frei von Histon 1 (H1) und Scl-70 ist, erhältlich durch ein Verfahren, bei dem man Zellkerne aus biologischem Material isoliert, man die Zellkerne enzymatisch mit einer Nuklease verdaut und durch Dichtegradientenzentrifugation mit einem Saccharose-Gradienten in Gegenwart von 0,55 mol/l NaCl aufreinigt, wobei man die Nukleosomen aus einer Fraktion mit mindestens 30 Gew.-% Saccharose isoliert.

Die Erfindung betrifft ferner ein Verfahren zur Herstellung einer Histon 1- (Hl-) und Scl-70-freien (und vorzugsweise einer nicht-Histon-Protein-freien) Nukleosomen-Präparation, bei dem man Zellkerne aus biologischem Material isoliert, man die Zellkerne enzymatisch mit einer Nuklease verdaut und durch Dichtegradientenzentrifugation mit einem Saccharose-Gradienten von bis zu 50 Gew.-% in Gegenwart von 0,55 mol/l NaCl aufreinigt, wobei man die Nukleosomen aus einer Fraktion mit mindestens 30 (vorzugsweise mindestens 35) Gew.-% Saccharose, vorzugsweise 30 bis 50 Gew.-% Saccharose isoliert, wobei ein Bereich von 35 (oder 40) bis 50 Gew.-% besonders bevorzugt ist. Am meisten bevorzugt ist, die Nukleosomen aus einer Fraktion mit etwa 50 Gew.-% Saccharose zu isolieren. Alternativ kann auch eine Gelfiltration durchgeführt werden. Dabei sind die Nukleosomen enthaltenden Fraktionen, wie unten beschrieben, daraufhin zu prüfen, ob insbesondere Scl-70 abgetrennt wurde oder noch in der Präparation vorhanden ist.

Die Erfindung betrifft ferner die Verwendung einer vorgenannten Nukleosomen-Präparation zum Nachweis von Systemischen lupus erythematodes (SLE) in vitro.

Erfindungsgemäß wird somit ein in vitro-Verfahren zur Diagnose von Systemischen lupus erythematodes (SLE) bereitgestellt, bei dem man eine flüssige Probe eines Patienten, wie z.B. Serum, mit Hilfe eines Antikörper-Nachweisverfahrens untersucht, bei dem man die erfindungsgemäßen Nukleosomen verwendet und man SLE dadurch diagnostiziert, daß man Antikörper gegen die Nukleosomen nachweist.

Dieser immunologische Nachweis wird mittels bekannter Methoden durchgeführt, wobei zur Detektion der Autoantikörper gegen Nukleosomen gemäß der Erfindung jedes beliebige direkte oder indirekte Verfahren zur Anwendung kommen kann. Dabei sind sowohl Flüssigphasenimmunassays als auch Festphasenimmunoassays denkbar [vgl. auch Price, C.P. und Newman, D.J., a.a.O. Principles and Practice of Immunoassay, 2nd Ed., 1997, Stockton Press, New York].

Im Gegensatz zu den indirekten Verfahren wird bei den direkten Nachweisen die Bindung der Antikörper an das Antigen über eine Änderung der chemischen oder physikalischen Eigenschaften bestimmt, so daß nachfolgende Detektionsschritte mit markierten Bindungspartnern entfallen.

Erfindungsgemäß bevorzugt erfolgt der Nachweis der Autoantikörper gegen die Nukleosomen in einem Immunoassay, bevorzugt in einem Festphasenimmunoassay, unter direkter oder indirekter Kopplung eines Reaktionspartners mit einer gut nachweisbaren Markierungssubstanz. Besonders bevorzugt kann der Nachweis in einem ELISA, einem RIA oder einem Fluoreszensimmunoassay erfolgen. Die Durchführung dieser Nachweisverfahren ist dem Fachmann gut bekannt.

In einem ELISA (enzyme linked immunosorbent assay) der vorliegenden Erfindung werden die Nukleosomen direkt oder indirekt an eine Trägersubstanz (z.B. Polystyrol) gebunden. Nach Inkubation mit den nachzuweisenden Antikörpern, z. B. aus dem Serum des Patienten, werden Antigen-gebundene Antikörper mittels Substanzen nachgewiesen. Diese Substanzen können Antikörper, Fragmente von Antikörpern oder hochaffine Liganden wie z.B. Avidin, das eine Biotin-Markierung bindet, sein. Als Enzyme kommen beispielsweise die Peroxidase, alkalische Phosphatase, β-Galaktosidase, Urease oder Glucoseoxidase in Betracht. Durch Zugabe eines chromogenen Substrats können die gebundenen Enzyme und damit beispielsweise die gebundenen Nukleosomen-Antikörper quantifiziert werden.

Auch in einem Radio-Immunoassay (RIA) ist das Antigen der Erfindung, die Nukleosomen, direkt oder indirekt an eine Trägersubstanz, wie etwa Polystyrol, gebunden. Nach Inkubation mit den nachzuweisenden Antikörpern, z.B. aus dem Serum von Patienten, werden die an das Antigen gebundenen Antikörper mit Hilfe von Substanzen nachgewiesen, die eine radioaktive Markierung tragen, z.B. ¹²⁵I. Diese Substanzen können ähnlich wie bei dem ELISA Antikörper, Fragmente von Antikörpern oder hochaffine Liganden wie z.B. Avidin, das eine Biotin-Markierung bindet, sein. Der quantitative Nachweis der an das Antigen gebundene Antikörper erfolgt über die gebundene Radioaktivität mittels eines geeigneten Meßgerätes.

Im erfindungsgemäßen Fluoreszenzimmunoassay (FIA) erfolgt der Nachweis von Antikörpern gegen die Nukleosomen ähnlich wie im RIA. Die Markierung in diesem Testsystem besteht aus einer Floureszenz-Markierung, wie z.B. Flourescein-Isothiocyanate (FITC). Auch hier kann ein quantitativer Nachweis der an das Antigen gebundenen Antikörper über den Nachweis des gebundenen Fluoreszenzfarbstoffes mittels eines geeigneten Meßgerätes erfolgen.

Denkbar ist im Rahmen der vorliegenden Erfindung auch der Nachweis von Antikörpern gegen die Nukleosomen über einen Agglutinationstest oder Geldiffusionstest. Beim Agglutinationstest werden Antigen-tragende Partikel z.B. Latex oder Polystyrol-Kügelchen durch Antikörper, z.B. aus dem Serum, quervernetzt. Die dadurch gebildeten Aggregate lassen sich turbodimetrisch nachweisen.

Beim Geldiffusionstest werden in einer Agar- oder Agarose-Gel-Platte benachbarte Vertiefungen mit dem Antigen z. B. Nukleosomen und dem Antikörper, z.B. Patientenserum gefüllt. Diffundieren die Substanzen aus den Vertiefungen, bilden sich Konzentrationsgradienten. In den überlappenden Bereichen bilden sich gut sichtbare Präzipitate, sobald die Konzentrationsverhältnisse zwischen Antigen und Antikörper ein Optimum erreicht und vorausgesetzt, daß Antikörper gegen das Antigen in der Antikörperlösung vorhanden sind.

Gemäß einer bevorzugten Ausführungsform wird ein in vitro-Verfahren zur SLE-Diagnose bereitgestellt, bei dem man eine flüssige Probe eines Patienten, wie z.B. Patientenserum, mit Hilfe eines ELISA untersucht, bei dem die Mikrotiterplatten mit erfindungsgemäßen Nukleosomen beschichtet sind und man SLE dadurch diagnostiziert, daß man in der Probe Antikörper gegen die Nukleosomen nachweist.

Gemäß einer besonderen Ausführungsform der Erfindung handelt es sich bei der Mikrotiterplatte um eine Mikrotiterplatte, bei der mindestens ein Teil der Kavitäten (alternativ: alle Kavitäten) mit den Nukleosomen der vorliegenden Erfindung beschichtet ist (sind).

Gegenstand der vorliegenden Erfindung sind neben der erwähnten Mikrotiterplatte auch andere feste Träger, die mit den erfindungsgemäßen Nukleosomen beschichtet sind bzw. auf deren Oberfläche die Nukleosomen fixiert sind (s.o.), wie z.B. Nitrocellulose oder PVDF (Polyvinylidenfluorid).

Gemäß einer weiteren Ausführungsform betrifft die Erfindung ein in vitro-Verfahren zu SLE-Diagnose, bei dem man einen Streifentest (z.B. EUROLINE^{®}; vgl. Price, C.P. und Newman, D.J., a.a.O.) unter Verwendung Nukleosomen-beschichteter Nitrocellulose oder PVDF durchführt.

Die Erfindung betrifft ferner ein Kit zur Durchführung eines genannten *in vitro*-Verfahrens zur Diagnose von Systemischen lupus erythematodes, das eine erfindungsgemäße Nukleosomen-Präparation, einen oben genannten festen Träger oder eine oben genannte Mikrotiterplatte, Nitrocellulose oder PVDF enthält. Gegebenenfalls können in dem Kit weitere zur Durchführung des Antikörper-Nachweisverfahrens erforderliche oder nützliche Reagenzien und Hilfsmittel enthalten sein.

Die Erfindung wird nachfolgend anhand von Beispielen und Figuren beschrieben:

### Beispiele

### Isolierung von Nukleosomen aus Kalbsthymus

### Konventionell Nukleosomen

### Extraktion des Kalbsthymus

5g Kalbsthymus [alternativ können auch Zellen einer anderen Quelle verwendet werden, üblicherweise von tierischen Geweben (humane eher weniger), wie z.B. Rattenleber, Kaninchenthymus etc., oder tierische Zellen, wie Hühner-Erythrocyten, und Zellkulturen (z.B. mouse erythroleukemia L1210 cells)] werden in 150 mL 0,9%iger NaCl-Lösung aufgenommen und zerkleinert. Anschließend wird die Suspension homogenisiert. Nach Abfiltrieren der groben Bestandteile wird die Suspension zentrifugiert (15 Min bei 1.100 x g, 4°C). Das Pellet wird in jeweils 30 mL 0,9%ige NaCl-Lösung zwei mal gewaschen wie oben zentrifugiert. Anschließend wird das Pellet zur Kern-Aufreinigung weiterverarbeitet

### Kern-Aufreinigung

Die Kern-Aufreinigung erfolgt nach Subirana [Subirana, JA. Studies on the thermal denaturation of nucleohistones. J Mol Biol. 1973, 74, 363-86]. Das Pellet wird in 40 mL Saccharose-1-Lösung (250 mM Saccharose, 3 mM CaCl₂·2 H₂O) resuspendiert und anschließend zentrifugiert (12 Min, 5.000 Upm und 4°C)**.** Nach Aufnahme des Pellets in 45 mL Saccharose-2-Lösung (2 M Saccharose, 3 mM CaCl₂·2 H₂O, 0,5 % Triton-X-100) wird wieder zentrifugiert (22 Min, 13.200 x g und 4°C). Das Pellet wird in 45 mL Puffer 1 (0,1 M Tris/HCl pH 8, 0) resuspendiert und wiederum zentrifugiert (15 Min, 1.100 x g, 4°C). Der letze Waschschritt wird wiederholt. Das Pellet wird in 45 mL 0,9%ige NaCl-Lösung aufgenommen und die Lösung zentrifugiert (15 Min, 1.100 x g, 4°C). Der Überstand wird verworfen und das Zellkern-enthaltene Pellet weiterverarbeitet.

### Verdau der Kerne

Das Pellet wird in 40 mL Puffer 2 (15 mM Tris/HCl, pH 7, 5, 15 mM NaCl, 60 mM KNO₃, 0,25 M saccharose, 5 mM MgCl₂·6 H₂O, 1 mM CaCl₂-2 H₂O, 1 mM DTT) ausgenommen und Suspension homogenisiert. Dann werden 750 Units der Nuklease S7-Lösung zur Suspension hinzu gefügt, die Lösung 30 Min im Wasserbad bei 37°C inkubiert und danach zentrifugiert (15 Min, 4.000 Upm, 4°C). Das entstandene Pellet wird in 10 mL 2 mM-EDTA-Lösung aufgenommen. Die Suspension wird 45 Min unter Schütteln bei Raumtemperatur inkubiert und anschließend zentrifugiert (15 Min, 4.000 Upm, 4°C). Schließlich wird der Überstand vorsichtig abgenommen und als konventionelle. Nukleosom-Präparation eingesetzt.

### Nukleosomen Präparation gemäß Erfindung

Gemäß der Erfindung werden die Nukleosomen proteinabgereichert, das Resultat sind im wesentlichen Nukleoaomenkerne, die besonders als Antigene in der in-vitro-Diagnostik des SLE ihre Anwendung finden. Hier wird die Herstellung an einem Beispiel beschrieben.

### Nuklease-Abbau

Die konventionelle Nukleosom-Präparation wird über Nacht gegen Puffer 2 dialysiert. Anschließend wird die Lösung gekühlt (0°C) und unter Schütteln eine NaCl-Lösung in die Nukleosomen-Präparation getropft, bis eine Endkonzentration von 0,55 M NaCl erreicht wird. Schließlich werden 150 Units Nuklease S7 pro mL Nukleosom-Lösung hinzugefügt, die Lösung 30 Min im Wasserbad bei 37°C inkubiert und danach wieder auf 0°C abkühlt.

### A) Saccharose-Gradient (1. Methode)

Die erhaltenen Nukleosomen werden durch einen Dichtegradienten-Zentrifugation aufgereinigt: Jeweils 25 mL 50%, 30% und 10% Saccharose-Lösungen in TE-Puffer (10 mM Tris, 1 mM EDTA, 0,55 M NaCl) werden in ein Ultrazentrifugenröhrchen geschichtet und 5 mL Nukleosom-Lösung aufgetragen. Der Gradient wird über Nacht bei 4°C und 140.000 x g zentrifugiert.

Alternativ können auch andere Gravdienten verwendet werden, wie z.B. 50/40/30/10% oder 50/35/10% usw.

Die proteinabgereichten Nukleosomen befinden sich in der mindestens 30% Saccharose, vorzugsweise in der mindestens 35% (oder 40%) bzw. in der etwa 50% Saccharose enthaltenden Fraktion.

### B) Gelfiltration (2. Methode)

Die Isolierung der Nukleosomen kann ebenso anstelle der Saccharose-Dichte-zentrifugation über eine Gelfiltration (HiPrep 26/60 Sephacryl S-300 HR, Amersham-Bioscience) erfolgen. 10 mL der erhaltenen Nukleosomen werden auf die mit TE-Puffer äquilibrierte Säule aufgetragen und bei einer Elutionsgeschwindigkeit von 1 ml/min eluiert (TE-Puffer). Die Nukleosomen enthaltenden Fraktionen werden vereinigt.

### Charakterisierung der Nukleosomen

### ELISA-Studien: Spezifität von Nukleosomen als Antoantigen für SLE

Mittels ELISA wurden Studien mit Seren von Patienten mit SLE und progressiver Systemsklerose durchgeführt, welche die These unterstützen, daß die klinische Spezifität bei Verwendung der Nukleosomen als Autoantigen für SLE auf 100% steigen kann. Voraussetzung dafür ist, daß die Nukleosomen erfindungsgemäß soweit isoliert werden, bis mittels Silberfärbung nach SDS-PAGE der Nukleosomen keine weiteren Proteine mehr nachweisbar sind.

Es wurden zwei ELISA durchgeführt, bei denen verschiedene Nukleosomen-Präparationen zur Beschichtung der Mikrotiterplatte verwendet wurden.

### ELISA-Beschichtung

Die Mikrotiterplatten werden mit in PBS verdünnten Nukleosomen über Nacht bei Raumtemperatur beschichtet. Anschließend werden die mit Antigen beschichteten Platten mit PSS/0,1% Tween gewaschen und für 2 Stunden bei Raumtemperatur mit PBS-10% Fetalem Rinderserum geblockt. Danach werden die Mikrotiterplatten erneut mit PBS-0.1% Tween gewaschen. Die Inkubation der Platten erfolgt wie in den EUROIMMUN-Arbeitsanleitungen beschrieben.

Die beiden, im ELISA eingesetzten Präparationen unterscheiden sich dadurch, daß einerseits die konventionelle Nukleosomen-Präparation angesetzt wurde, andererseits die Nukieosomen-Präparation, die einer weiteren Aufreinigung unterzogen wurde, um weitere Proteine zu entfernen und damit die Nukleosomen gemäß der Erfindung zu erhalten:

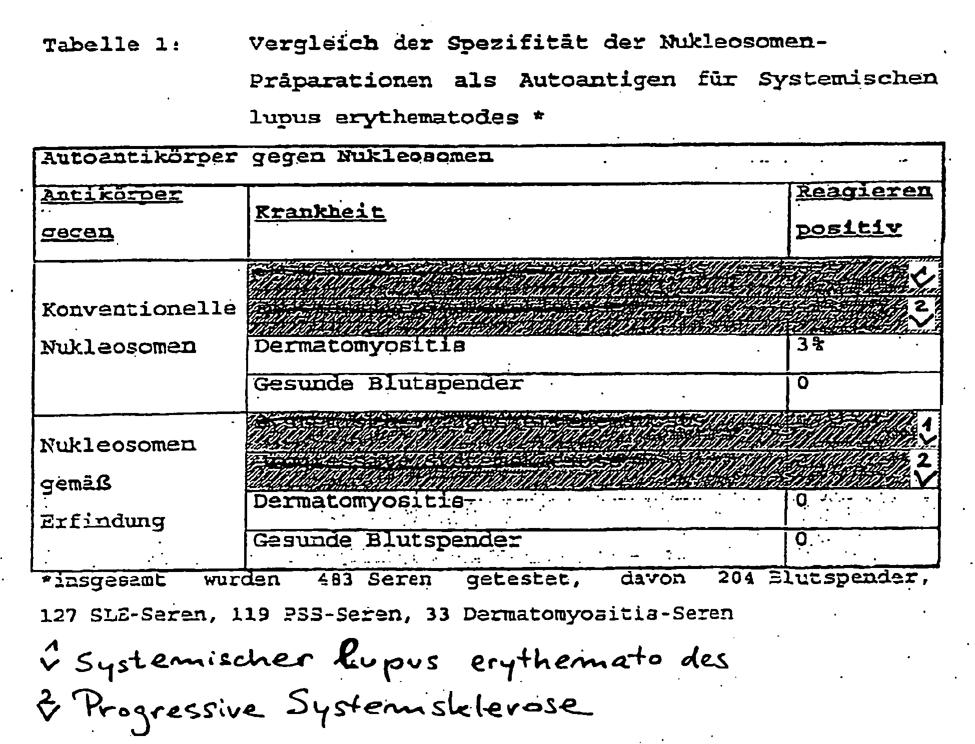

### SDS-PAGE

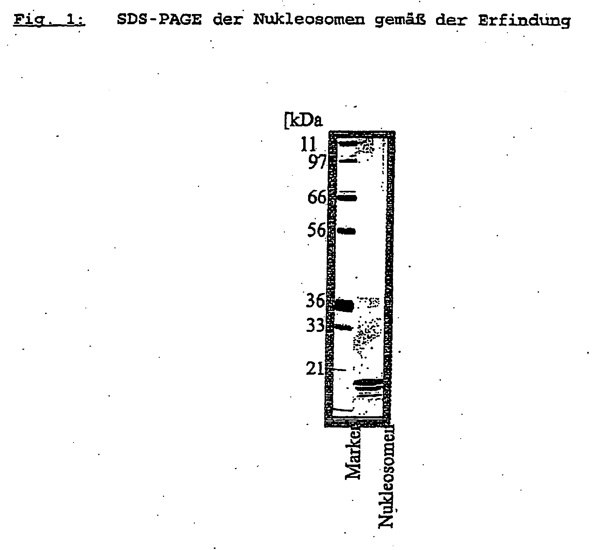

Die Nukleosomen gemäß der Erfindung wurden mittels SPS-PAGE mit anschließender Silberfärbung untersucht [alle Materalien für die Durchführung der Elektrophoresen stammen von Invitrogen: NuPAGE 4-12% Bis-Tris-Gel, NuPage Sample-Buffer, NuPage-MES-Laufpuffer, Mark-12. Anschließend wurde eine Silberfärbung nach Heukeshofen durchgeführt]. Das elektrophoretische Verhalten der Nukleosomen ist in Abb. 1 dargestellt. Das SDS-PA-Gel der Nukleosomen-Präparation gemäß der Erfindung zeigt keine Proteinbande oberhalb von 21 kDa, d.h., daß neben den im Nukleosomen-Kern befindlichen Histonen H2A, H2B, H3 und H4 keine weiteren Proteine in dieser Präparation nachweisbar sind.

### TAE-Agarose-Gel-Elektrophoresen

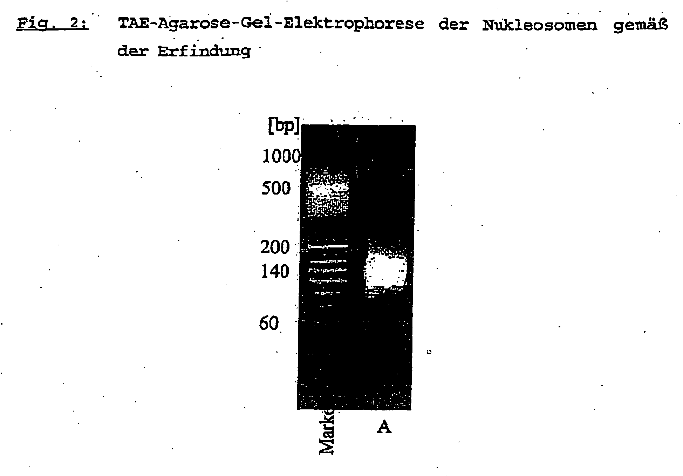

Die Größe der DNS-Fragmente in der Nukleosomen-Präparation wurde anhand von nativen 4%igen Tris-Acetat-EDTA-Agarose-Gel-Elektrophoresen bestimmt. Die Proben wurden zur Extraktion der Proteine der Wizard-DNA-Clean up unterzogen. Das Agarose-Gel der Nukleosomen (Fig. 2.) zeigt, daß in der Nukleosomenpräparation nur Mononukleosomen-DNS mit 150 Besenpaaren vorliegt. Höberkettige Chromatinreste sind nicht nachweisbar. [Hinsichtlich der Durchführung dieser Elektrophoresemethode wird auf folgende Publikation verwiesen: Hans Günter Gassen, Gangolf Schrimpf (Hrsg.): Gentechnische Methoden, 2. Auflage, 1999, Spektrum Akademischer Verlag, Heidelberg, Berlin, die Färbung des Agarose-Gels erfolgt Mittels Ethidiumbromid.]

### Westernblot

### Nachweis von Histon 1

Das Protein Histon 1 befindet sich im Gegensatz zu den Histonen 2A, 2B, 3 und 4 außen an den Nukleosomen. Das Molekulargewicht von Histon 1 beträgt etwa 21 kDa, jedoch zeigt dieses Protein aufgrund seiner stark positiven Landung ein verändertes Migrationsverhalten in der SDS-Gelelektrophorese: es erscheint als Bande bei ca. 33 kDa.

Histon 1 spielt als ein Autoantigen bei der rheumatoiden Arthritis eine große Rolle. Um Kreuzreaktionen auszuschließen und die Spezifität der Nukleosomen für SLE zu erhalten, muß Histon 1 von der Nukleosomen-Präparation abgetrennt werden. Dies ist durch die oben beschriebenen Reinigungsmethoden möglich.

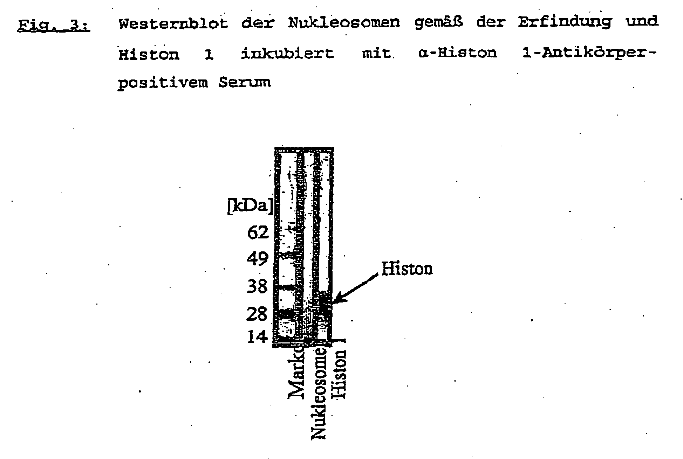

Nukleosomen gemäß der Erfindung wurden hinsichtlich ihrer Kontamination mit Histon 1 mit Hilfe von Westernblots untersucht. Auf das SDS-PA-Gel wurden die Nukleosomen gemäß der Erfindung und gereinigtes Histon 1 aufgetragen. Das Gel wurde auf Nitrocellulose geblottet und nach dem Blotten mit α-Histon-1-Antikörper-positivem Serum inkubiert. Anhand dieses Westernblots (Fig. 3) kann gezeigt werden, daß die Nukleosomen gemäß der Erfindung frei von Histon 1 sind.

### Nachweis von Scl-70

Scl-70 ist ein spezifisches Antigen für die Diagnose von progressiver Systemsklerose (Prävalenz: 25-70%). Es handelt bei diesem Antigen um das Protein DNS-Topoisomerase I. Dieses Enzym ist an der Reaktion, die mit einer Änderung der DNS-Topologie einhergeht, beteiligt. Das Molekulargewicht beträgt 100 kDa, jedoch reagieren die Antikörper der PSS-Seren auch mit dem 70 kDa-Abbauprodukt der DNS-Topoisomerase I, daher auch der Name Scl-70. Wie in Tabelle 1 ersichtlich ist, weist der ELISA beschichtet mit den konventionellen Nukleosomen eine Sensitivität für PSS von 41% auf, dagegen zeigt der mit den Nukleosomen gemäß der Erfindung beschichtete ELISA keine Reaktion mehr mit den PSS-Seren. Dieser Effekt läßt sich darauf zurückführen, daß durch die oben beschriebene Präparation der Nukleosomen gemäß der Erfindung das Scl-70 nahezu quantitativ abgetrennt wurde.

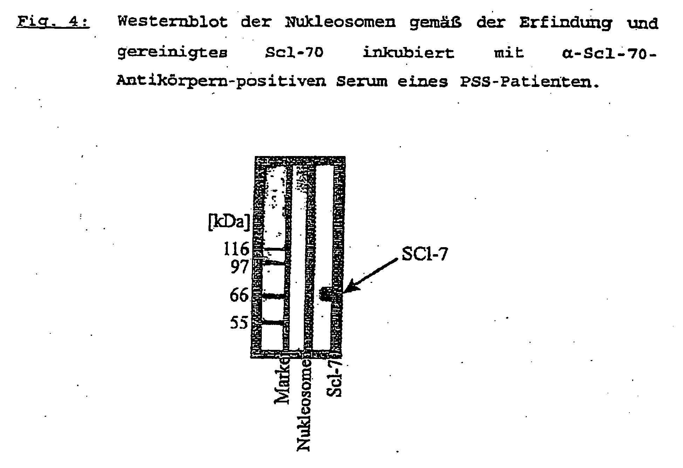

Mittels Westernblots wurden die Nukleosomen gemäß der Erfindung hinsichtlich ihrer Kontamination mit Scl-70 untersucht. Auf das SDS-PA-Gel wurden die Nukleosomen und isoliertes Scl-70 aufgetragen, nach der Elektrophorese wurden die Proteine auf Nitrocellulose geblottet und mit einem α-Scl-70-Antikörpern-positivem Serum inkubiert. Der in Fig. 4 gezeigte Immunoblot zeigt eindeutig, daß die Nukleosomen gemäß der Erfindung frei von Scl-70 sind.

### Zusammenfassung der Ergebnisse:

Anhand von ELISA Studien zeigt sich, daß die Nukleosomen gemäß der Erfindung spezifisch für SLE sind.

Die Präparation der Nukleosomen gemäß der Erfindung besteht nur aus Mononukleosomen, höberkettige Chromatinreste sind nicht nachweisbar. Die Untersuchung der Nukleosomen gemäß der Erfindung mittels SDS-PAGE mit anschließender Silberfärbung weist nur Proteine mit einer Molekularmasse unterhalb 21 kDa auf. Anhand von Immunoblots konnte gezeigt werden, daß die Nukleosomen frei von H1 und Scl-70 sind. Vorzugsweise enthalten sie ferner keine anderen nicht-Histon-Proteine, wie z.B. HMGN.

## Patentansprüche

1. Nukleosomen-Präparation, **dadurch gekennzeichnet, daß** sie frei von Histon 1 und Scl-70 ist erhältlich durch ein Verfahren, bei dem man Zellkerne aus biologischem Material isoliert, man die Zellkerne enzymatisch mit einer Nuklease verdaut und durch Dichtegradientenzentrifugation mit einem Saccharose-Gradienten in Gegenwart von 0,55 mol/l NaCl aufreinigt, wobei man die Nukleosomen aus einer Fraktion mit mindestens 30 Gew.-% Saccharose isoliert.

2. Nukleosomen-Präparation nach Anspruch 1, **dadurch gekennzeichnet, daß** sie Nukleosomen enthält, die aus einer Fraktion mit mindestens 40 Gew.-% Saccharose isoliert sind.

3. Nukleosomen-Präparation nach Anspruch 2, **dadurch gekennzeichnet, daß** sie Nukleosomen enthält, die aus einer Fraktion mit etwa 50 Gew.-% Saccharose isoliert sind.

4. Verfahren zur Herstellung einer Histon 1- und Scl-70-freier Nukleosomen-Präparation, bei dem man Zellkerne aus biologischem Material isoliert, man die Zellkerne enzymatisch mit einer Nuklease verdaut und durch Saccharose-Dichtegradientenzentrifugation aufreinigt, **dadurch gekennzeichnet, daß** man mit einem Saccharose-Gradienten in Gegenwart von 0,55 mol/l NaCl aufreinigt, wobei man die Nukleosomen aus einer Fraktion mit mindestens 30 Gew.-% Saccharose isoliert.

5. Verfahren nach Anspruch 4, **dadurch gekennzeichnet, daß** man die Nukleosomen aus einer Fraktion mit mindestens 40 Gew.-% Saccharose isoliert.

6. Verfahren nach Anspruch 5, **dadurch gekennzeichnet, daß** man die Nukleosomen aus einer Fraktion mit etwa 50 Gew.-% Saccharose isoliert.

7. Verwendung einer Nukleosomen-Präparation nach den Ansprüchen 1 bis 3 zum Nachweis von Systemischem lupus erythematodes (SLE) in vitro.

8. In vitro-Verfahren zur Diagnose von Systemischem lupus erythematodes (SLE), bei dem man eine flüssige Probe eines Patienten mit Hilfe eines Antikörper-Nachweisverfahrens untersucht, bei dem man Nukleosomen nach den Ansprüchen 1 bis 3 verwendet und man SLE **dadurch** diagnostiziert, daß man Antikörper gegen die Nukleosomen nachweist.

9. Verfahren nach Anspruch 8, **dadurch gekennzeichnet, daß** das Antikörper-Nachweisverfahren ein ELISA ist, bei dem die Mikrotiterplatte des ELISA mit Nukleosomen nach den Ansprüchen 1 bis 3 beschichtet ist.

10. Verfahren nach Anspruch 8, **dadurch gekennzeichnet, daß** das Antikörper-Nachweisverfahren ein Streifentest ist, bei dem ein fester Träger mit Nukleosomen nach den Ansprüchen 1 bis 3 beschichtet ist.

11. Verfahren nach den Ansprüchen 8 bis 10, **dadurch gekennzeichnet, daß** die flüssige Probe Serum ist.

12. Fester Träger, **dadurch gekennzeichnet, daß** er mit Nukleosomen nach den Ansprüchen 1 bis 3 beschichtet ist.

13. Träger nach Anspruch 12, **dadurch gekennzeichnet, daß** er Nitrocellulose oder Polyvinylidenfluorid ist.

14. Mikrotiterplatte, **dadurch gekennzeichnet, daß** mindestens ein Teil der Kavitäten mit Nukleosomen nach den Ansprüchen 1 bis 3 beschichtet ist.

15. Kit zur Durchführung des Verfahrens nach einem der Ansprüchen 8 bis 11, **dadurch gekennzeichnet, dass** es eine Nukleosomen-Präparation nach den Ansprüchen 1 bis 3, einen festen Träger nach Anspruch 12 oder 13 oder eine Mikrotiterplatte nach Anspruch 14 umfasst.

16. Kit nach Anspruch 15, **dadurch gekennzeichnet, daß** er weitere zur Durchführung des Antikörper-Nachweisverfahrens erforderliche oder nützliche Reagenzien und Hilfsmittel enthält.

## Claims

1. Nucleosome preparation **characterised in that** it is free from histone 1 and Scl-70, obtainable by a process in which cell nuclei are isolated from biological material, the cell nuclei are enzymatically digested with a nuclease and purified by density gradient centrifuging with a saccharose gradient in the presence of 0.55 mol/l NaCl, the nucleosomes being isolated from a fraction with at least 30% by weight of saccharose.

2. Nucleosome preparation according to claim 1 **characterised in that** it contains nucleosomes which are isolated from a fraction with at least 40% by weight of saccharose.

3. Nucleosome preparation according to claim 2 **characterised in that** it contains nucleosomes which are isolated from a fraction with approximately 50% by weight of saccharose.

4. Method for producing a nucleosome preparation free from histone 1 and Scl-70 in which the cell nuclei are isolated from biological material, the cell nuclei are digested enzymatically with a nuclease and purified by saccharose density gradient centrifuging **characterised in that** the purification is carried out with a saccharose gradient in the presence of 0.55 mol/l NaCl, the nucleosomes being isolated from a fraction with at least 30% by weight of saccharose.

5. Process according to claim 4 **characterised in that** the nucleosomes are isolated from a fraction with at least 40% by weight of saccharose.

6. Process according to claim 5 **characterised in that** the nucleosomes are isolated from a fraction with approximately 50% by weight of saccharose.

7. Use of a nucleosome preparation according to claims 1 to 3 for the detection of systemic lupus erythematosus (SLE) in vitro.

8. In vitro process for the diagnosis of systemic lupus erythematosus (SLE), in which a liquid sample from a patient is examined by means of an antibody detection process in which nucleosomes according to claims 1 to 3 are used and SLE is diagnosed by detecting antibodies to the nucleosomes.

9. Process according to claim 8 **characterised in that** the antibody detection process is an ELISA in which the microtitre plate of the ELISA is coated with nucleosomes according to claims 1 to 3.

10. Process according to claim 8 **characterised in that** the antibody detection method is a line assay in which a solid carrier is coated with nucleosomes according to claims 1 to 3.

11. Process according to claims 8 to 10 **characterised in that** the liquid sample is serum.

12. Solid carrier **characterised in that** it is coated with nucleosomes according to claims 1 to 3.

13. Carrier according to claim 12 **characterised in that** it is nitrocellulose or polyvinylidene fluoride.

14. Microtitre plate **characterised in that** at least some of the cavities are coated with nucleosomes according to claims 1 to 3.

15. Kit for the execution of the process according to claims 8 to 11, **characterised in that** it comprises a nucleosome preparation according to claims 1 to 3, a solid carrier according to claim 12 or 13 or a microtitre plate according to claim 14.

16. Kit according to claim 15 **characterised in that** it comprises further reagents and auxiliary agents necessary or useful for the execution of the method for detection of antibodies.

## Revendications

1. Préparation de nucléosomes, **caractérisée en ce qu'**elle est exempte d'histone 1 et de Scl-70, pouvant être obtenue par un procédé dans lequel on isole des noyaux cellulaires à partir d'une matière biologique, on fait digérer par voie enzymatique les noyaux cellulaires à l'aide d'une nucléase et on les purifie par centrifugation en gradient de densité, avec un gradient de saccharose, en présence de 0,55 mole/l de NaCl, de sorte qu'on isole les nucléosomes à partir d'une fraction comportant au moins 30 % en poids de saccharose.

2. Préparation de nucléosomes selon la revendication 1, **caractérisée en ce qu'**elle contient des nucléosomes qui sont isolés à partir d'une fraction comportant au moins 40 % en poids de saccharose.

3. Préparation de nucléosomes selon la revendication 2, **caractérisée en ce qu'**elle contient des nucléosomes qui sont isolés à partir d'une fraction comportant environ 50 % en poids de saccharose.

4. Procédé pour la production d'une préparation de nucléosomes exempte d'histone 1 et de Scl-70, dans lequel on isole des noyaux cellulaires à partir d'une matière biologique, on fait digérer par voie enzymatique les noyaux cellulaires à l'aide d'une nucléase et on les purifie par centrifugation en gradient de densité de saccharose, **caractérisé en ce qu'**on purifie à l'aide d'un gradient de saccharose en présence de 0,55 mole/l de NaCl, de sorte qu'on isole les nucléosomes à partir d'une fraction comportant au moins 30 % en poids de saccharose.

5. Procédé selon la revendication 4, **caractérisé en ce qu'**on isole les nucléosomes à partir d'une fraction comportant au moins 40 % en poids de saccharose.

6. Procédé selon la revendication 5, **caractérisé en ce qu'**on isole les nucléosomes à partir d'une fraction comportant environ 50 % en poids de saccharose.

7. Utilisation d'une préparation de nucléosomes selon les revendications 1 à 3, pour la détection du lupus érythémateux disséminé (LED) in vitro.

8. Procédé in vitro pour le diagnostic du lupus érythémateux disséminé (LED), dans lequel on examine un échantillon liquide d'un patient, à l'aide d'un procédé de détection au moyen d'anticorps, dans lequel on utilise des nucléosomes selon les revendications 1 à 3, et on diagnostique le LED par le fait qu'on détecte des anticorps dirigés contre les nucléosomes.

9. Procédé selon la revendication 8, **caractérisé en ce que** le procédé de détection au moyen d'anticorps est un dosage ELISA, dans lequel la plaque de microtitrage du dosage ELISA est tapissée avec des nucléosomes selon les revendications 1 à 3.

10. Procédé selon la revendication 8, **caractérisé en ce que** le procédé de détection au moyen d'anticorps est un procédé utilisant des bandes, dans lequel un support solide est revêtu avec des nucléosomes selon les revendications 1 à 3.

11. Procédé selon les revendications 8 à 10, **caractérisé en ce que** l'échantillon liquide est du sérum.

12. Support solide, **caractérisé en ce qu'**il est revêtu avec des nucléosomes selon les revendications 1 à 3.

13. Support selon la revendication 12, **caractérisé en ce qu'**il est en nitrocellulose ou poly(fluorure de vinylidène).

14. Plaque de microtitrage, **caractérisée en ce qu'**au moins une partie des cavités est revêtue avec des nucléosomes selon les revendications 1 à 3.

15. Nécessaire pour la mise en oeuvre du procédé selon l'une quelconque des revendications 8 à 11, **caractérisé en ce qu'**il comprend une préparation de nucléosomes selon les revendications 1 à 3, un support solide selon la revendication 12 ou 13, ou une plaque de microtitrage selon la revendication 14.

16. Nécessaire selon la revendication 15, **caractérisé en ce qu'**il contient d'autres réactifs et adjuvants utiles ou nécessaires à la mise en oeuvre du procédé de détection au moyen d'anticorps.
